# EUROPEAN PATENT APPLICATION

(11) **EP 4 230 206 A1**
(43) Date of publication of application: **23.08.2023**
(21) Application number: 20923687.6
(22) Date of filing: 15.10.2020
(51) Int. Cl.: A61K 31/485, A61K 47/36, A61P 11/02

(54) **PHARMACEUTICAL COMPOSITION BASED ON NALBUPHINE AND/OR ITS SALTS FOR NASAL ADMINISTRATION**

(71) Applicant: Pharmbiotest Poland SP.ZO.O., 86-300 Grudziadz (PL)
(72) Inventor: TYMKO, Volodymyr, Lugansk region, 93012 (UA); OLIINYKOV, Dmytro, Lugansk region, 93010 (UA); CHORNYI, Oleksandr, Lugansk region, 93010 (UA)
(74) Representative: Augustyniak, Magdalena Anna
(86) International application number: PCT/IB2020/059683
(87) International publication number: WO 2022/038403

(57) **Abstract**

The present disclosure relates generally to the pharmaceutical field, and more particularly to a stable pharmaceutical composition for nasal administration comprising a therapeutically effective amount of nalbuphine and/or salts thereof.

The essence of the present invention consists in that the pharmaceutical composition comprises:
- an active pharmaceutical ingredient for which nalbuphine as the base and/or salts thereof are used,
- an enhancer of the adsorption of said active pharmaceutical ingredient with chitosan and/or derivatives thereof including in the form of chitosan salts being used as said adsorption enhancer,
- a solvent of said active pharmaceutical ingredient and said adsorption enhancer,
wherein the amount of the active pharmaceutical ingredient being 2.5 to 20 % *w*/*w* and the active pharmaceutical ingredient to adsorption enhancer ratio being from 1:0.1 through 1:1.

The technical result of the present invention is the provision of a nasal pharmaceutical composition based on nalbuphine and/or salts thereof having a high bioavailability of nalbuphine to treat (relieve) moderate to severe pain.

## Description

### Field of the Invention

The present disclosure relates generally to the pharmaceutical field, and more particularly to a nasal pharmaceutical composition comprising a therapeutically effective amount of nalbuphine and/or salts thereof.

The present invention is aimed at providing a nasal pharmaceutical composition based on nalbuphine and/or salts thereof to eliminate moderate to severe pain in traumas, during a postoperative period, as well as a basic or auxiliary pain medication in pain syndromes of various etiology.

### Description of the Prior Art

A pain syndrome (pain) is a core symptom of many diseases such as, for example, cancer, arthritis, neurological diseases, and heart attacks, among others. A pain syndrome is an integrative function of many body systems to protect the body against exposure to harmful factors. The pain syndrome affects consciousness, feelings, memory, motivation, vegetative mechanisms, behavioral reactions, and emotions. An incorrect or insufficient treatment for pain may result in mental disorders or neurological abnormalities, in the progression of underlying disease, and, in some extreme cases, in suicide attempts.

Known from the state of art are pharmaceutical compositions for systemic use based on opioid analgesics such as buprenorphine, tramadol, and nalbuphine, which are used to treat (relieve) moderate to severe pain. Buprenorphine is manufactured and used in the form of solutions for injection and transdermal therapeutic systems; tramadol is manufactured and used in the form of solutions for injection, oral droplets, capsules, tablets, and suppositories. Nalbuphine is only manufactured and used in the form of solutions for injection, which have the following inherent disadvantages: patient traumatization when administering the medicine; the need for a sterile syringe and healthcare personnel; the possibility of the exposure of patient to infections associated with providing health care services, especially in case of skin disorders; *etc.*

The major advantage of opioids is that they have an extensive history of use and are much more effective in treating severe pain than other classes of medications, e.g., aspirin, paracetamol, ibuprofen, *etc.* Furthermore, the opioid analgesics unlike NSAIDs (non-steroidal anti-inflammatory drugs), exert no serious adverse effects on the gastrointestinal tract (GIT) organs. In addition to relieving pain, opioids have other beneficial effects such as, for example, peripheral arterial vasodilation and, when treating heart attacks, provide the benefit of reducing oxygen demand on the heart.

Nalbuphine hydrochloride ('nalbuphine' for short) is (5α.6α)-17-(cyclobutylmethyl)-4.5epoxy-morphinan-3.6.14-triol hydrochloride with opiate agonist and antagonist effects. Nalbuphine was synthesized based on the potent analgesic oxymorphone and naloxone antagonist. Nalbuphine has a potent analgesic effect due to the agonistic effect on kappa-opiod receptors. Nalbuphine is also an antagonist of µ-receptors, and therefore does not have pronounced euphoria and drug dependence. When administered intramuscularly, nalbuphine is comparable in analgesic activity with morphine. Unlike morphine and fentanyl, nalbuphine does not significantly affect the pressure in the bile ducts and does not inhibit the motility of the gastrointestinal tract. In its general mode of action, nalbuphine is close to pentazocine but exerts a more potent analgesic action with weaker side effects and a lower ability of developing drug tolerance and opioid dependence.

Known from the state of art is a dosage form of nalbuphine in the form of a solution for injection in ampoules containing 10 and 20 mg of the medicine in 1 ml (go, for example, to: https://liki24.com/p/nalbufin-inekcii-20-mg-r-r-d-20-mgml-amp-1-ml-5-rusan-pharma/).

As was already mentioned, currently, nalbuphine is only commercially available in the form of a solution for injection. If nalbuphinea in the form of a solution for injection is used intramuscularly, its therapeutic effect lasts for about 3 to 4 hours, while following intravenous bolus administration of nalbuphine solution, its therapeutic effect lasts for about 1.5 to 2 hours. There have been attempts to create basal nalbuphine-based pharmaceutical compositions (Lo MW, Senary WL, Whitney CC Jr, The disposition and bioavailability of intravenous and oral nalbuphine in healthy volunteers., at J. Clin. Pharmacol. 1987 Nov;27(I I):866-73). Following nasal use (administration), the bioavailability of nalbuphine proves, however, to be very low (about 16.4 to 17.7 per cent) this being explained by its strong first-pass effect (for reference, go to https://ru.qwe.wiki/wiki/First_pass effect) 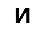 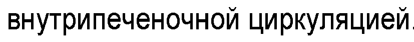

The nasal (intranasal) route of administration is currently receiving special interest, especially in the area of pain management. When a pharmaceutical composition in the form of spray is administered via the intranasal route, the active ingredient is applied to the nasal mucosa where it is absorbed. The extensive network of blood capillaries under the nasal mucosa is particularly suited to provide rapid and effective systemic absorption of a nasal pharmaceutical composition.

The intranasal administration of medication provides numerous advantages over both intravenous and intramuscular administration. The principal advantages of the intranasal administration include a non-invasive administration; a rapid absorption of the active pharmaceutical ingredient; usability; and the assurance of a rapid form of adjusting a medicine amount. Both intravenous and intramuscular routes of administration, unlike the intranasal administration, require sterility in the administration site; the availability of additional medical products, for example, syringes; as well as are often used only on admission to a healthcare institution and by skilled medical personnel. The entire procedure of medication administration (injection) is rather complicated and time-consuming.

In contrast, intranasal administration requires little time for the preparation and administration of a medicine and no complicated procedures.

A second important advantage of intranasal administration over intravenous and intramuscular administrations is patient acceptance of the intranasal delivery route. In some cases, the injections cause burning, edema, swelling, turgidity, hardness, and soreness at the injection site. In contrast, intranasal administration is perceived as non-invasive, is not accompanied by pain, has no after-effects, and produces rapid pain relief in the patient exhibiting pain symptoms. This is of particular advantage when the patient is a child.

The most important advantage of intranasal administration is no very high level of blood concentrations of the active pharmaceutical ingredient. Despite a rapid achievement of therapeutic effect and the duration thereof for several hours, intranasal administration of a medication causes no peak plasma concentrations of the active pharmaceutical ingredient which concentrations are responsible for the occurrence of side effects.

In the event of invasive administration of opioids, the maximum starting concentration of the active pharmaceutical ingredient may be several times therapeutic concentration required. On each next administration of the active pharmaceutical ingredient, the patient suffers from peak loads on the body, which manifest themselves as general weakness, dizziness, and other adverse effects, in some cases, loss of consciousness as well.

In contrast, an intranasal composition free of the above disadvantages makes it possible to administer the medication whenever required and to control (dose) the level required of plasma active pharmaceutical ingredient concentration without achieving peak values, and to achieve thereby a desired level of pain management.

Accordingly, what is needed is a nasal pharmaceutical composition based on nalbuphine and/or salts.

Known from the state of art are a nasal hydromorphone-based pharmaceutical composition (US8198291 B2, published on 12.06.2012) and a nasal fentanyl-based pharmaceutical composition (JP4898073 B2, published on 14.03.2012), characterized in that such pharmaceutical composition contains the active pharmaceutical ingredient (hydromorphone, fentanyl) and a solvent of said active pharmaceutical ingredient.

Nalbuphine, unlike these opioids (hydromorphone, fentanyl), is receiving special interest as a pain medication. It is comparable in analgesic activity with morphine but inhibits the respiratory center and affects the motility of the gastrointestinal tract to a lesser extent, and does not affect hemodynamic parameters.

The principal advantage of nalbuphine over the other opioids is the minimal risk of developing drug tolerance and opioid dependence if used in a controlled manner.

### Summary of the Invention

In view of the above-mentioned disadvantages of the prior art, it is an objective of the present invention to provide a nasal pharmaceutical composition based on nalbuphine and/or salts thereof, which has a high bioavailability of nalbuphine, to treat (relieve) moderate to severe pain.

Furthermore, it is an objective of the present invention to widen the arsenal of nasal opioid-based pharmaceutical compositions.

The other objectives and advantages of the present invention will be better understood by reference to the following description of embodiments of the invention taken in conjunction with the accompanying drawings.

In one embodiment, the present invention provides a nasal pharmaceutical composition based on nalbuphine and/or salts thereof characterized in that it comprises:
- an active pharmaceutical ingredient for which nalbuphine as the base and/or salts thereof are used,
- an enhancer of the adsorption of said active pharmaceutical ingredient with chitosan and/or derivatives thereof including in the form of chitosan salts being used as said adsorption enhancer,
- a solvent of said active pharmaceutical ingredient and said adsorption enhancer,
wherein the amount of the active pharmaceutical ingredient being 2.5 to 20 % *w*/*w* and the active pharmaceutical ingredient to adsorption enhancer ratio being from 1:0.1 through 1:1.

According to one aspect of the present invention, salts of nontoxic organic and nonorganic acids such as, for example, acetic acid, benzoic acid, citric acid, fumaric acid, glutamic acid, hydrobromic acid, hydrochloric acid, lactic acid, maleic acid, nitric acid, pantothenic acid, phosphoric acid, succinic acid, sulfuric acid, and tartaric acid, are used as nalbuphine salts.

According to another aspect of the present invention, the absolute bioavailability of the active pharmaceutical ingredient is at least 40 %.

According to yet another aspect of the present invention, pH of the nasal pharmaceutical composition based on nalbuphine and/or salts thereof is between 4.0 and 7.0.

According to one aspect of the present invention, hydrobromic acid, citric acid, lactic acid, acetic acid, malic acid, phosphoric acid, sodium hydroxide, trometamol, diethanolamine, tert-butylamine, tetrahydroxypropyl ethylenediamine (THPE), potassium carbonate, potassium hydroxide, sodium carbonate, sodium citrate, and sodium lactate, for example, are used as pH regulators.

According to another aspect of the present invention, the pharmaceutical composition comprises an enhancer of the solubility of the active pharmaceutical ingredient with solubilizers and/or surfactants being used as said solubility enhancer.

According to yet another aspect of the present invention, monoethyl ether of diethylene glycol, cyclodextrins, glyceryl monostearate, lecithin, poloxamer, polyvinyl pyrrolidone, polyethylene alkyl ethers, hydrogenated castor oil derivatives, polyoxyethylene stearates, citric acid and ascorbic acid, polysorbates, sorbitan esters, polyvinyl alcohol, benzalkonium chloride and lauryl sulphate, for example, are used as said solubilizers.

According to still another aspect of the present invention, polysorbates and macrogols, for example, are used as said surfactants, and poloxamers and povidones are used as said solubilizers.

According to one aspect of the present invention, the pharmaceutical composition comprises a microbiological stability preservative with, for example, methylparaben, propylparaben, sodium benzoate, benzyl alcohol, benzalkonium chloride, and chlorobutanol being used as said microbiological stability preservative.

According to another aspect of the present invention, water or its mixture with organic co-solvents is/are used as said solvent with, for example, ethanol, propylene glycol, glycerol, and polyethylene glycols being used as said co-solvents.

According to yet another aspect of the present invention, the pharmaceutical composition comprises a viscosity regulator with, for example, hydroxypropyl methylcellulose, hydroxypropyl cellulose, hydroxyethyl cellulose, methylcellulose, sodium carboxylmethylcellulose, and polyvinyl pyrrolidones of various grades, such as K15, K30, K60, and K90, being used as said viscosity regulator.

According to still another aspect of the present invention, the pharmaceutical composition comprises an osmolarity regulator with, for example, sodium metabisulfite, sodium bisulfite, ethylenediaminetetraacetic acid (EDTA) disodium salt, and ascorbic acid being used as said osmolarity regulator.

According to one aspect of the present invention, the osmotic pressure of the pharmaceutical composition is 300 to 700 mOsm/L.

According to another aspect of the present invention, the pharmaceutical composition comprises an antimicrobial agent with, for example, benzyl alcohol, benzoic acid and/or salts thereof, sorbates, benzalkonium chloride, phenylethyl alcohol, methylparaben, ethylparaben, propylparaben, and butylparaben being used as said antimicrobial agent.

According to yet another aspect of the present invention, the pharmaceutical composition comprises taste, color, and odor correctives.

In another embodiment, the present invention provides a method of using the nasal pharmaceutical composition based on nalbuphine and/or salts thereof characterized in that the nasal pharmaceutical composition is administered at a dose rate of between 3 and 20 mg.

In a preferred embodiment of the present invention, the content of the active pharmaceutical ingredient is 3.0 to 4.0 % w/w.

According to one aspect of the present invention, the pharmaceutical composition is isotonic or hypertonic one, i.e. has the osmotic pressure of 300 to 700 mOsm/L which is equal to or more than that of body fluids.

According to another aspect of the present invention, the pharmaceutical composition is preferably is neutral or weak acid one with pH of the ready-for-use pharmaceutical composition being preferably from 4.0 through 7.0.

According to yet another aspect of the present invention, the process of preparing the pharmaceutical composition and of closing the immediate packaging is preferably performed under an inert gas to improve the stability of the active pharmaceutical ingredient

### Brief Description of the Drawings

In the discussion of embodiments of the present invention, domain specific terminology is used. The present invention is not limited, however, by the terms accepted, and it is contemplated that each such term covers all the equipment meanings which operate in a similar manner and are employed to solve the same problems.

The expression "based on nalbuphine" shall mean nalbuphine as the base and/or nalbuphine salts.

The nasal pharmaceutical composition provided herein comprises chitosan and/or derivatives thereof as the adsorption enhancer. The chitosan derivatives comprise substantially salts of organic acids, such as, for example, lactic acid, glutamic acid, or succinic acid.

For a better understanding of the present invention, and to show more clearly how it may be carried into effect, reference will now be made, by way of example only, to the accompanying drawings, in which:
- **Fig. 1**: is a graphic representation of the mean concentrations of nalbuphine versus time (in direct coordinates);
- **Fig. 2**: is a comparative diagram of the adverse reactions recorded; and
- **Fig. 3**: is a graphic representation of the mean concentrations of nalbuphine versus time (in direct coordinates).

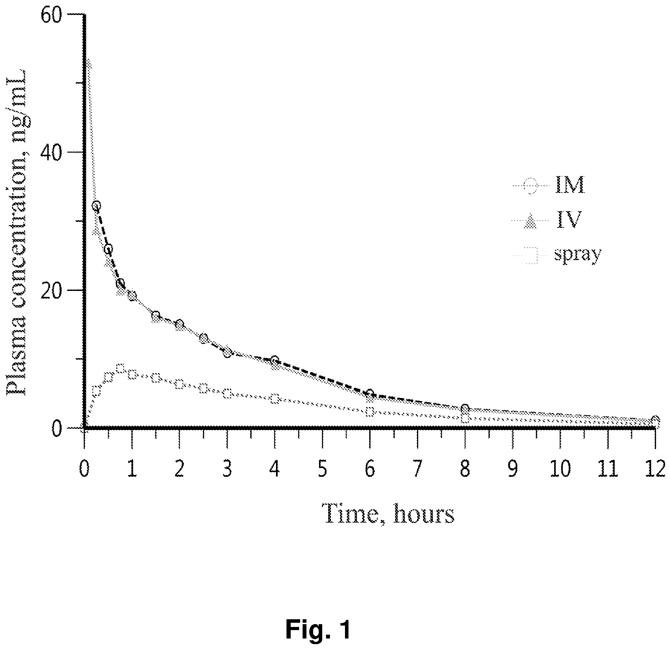

The list of legends, terms, and abbreviations given in **Figs. 1 to 3**
**A** - Medication A consisting of composition 2 (Example 2);
**B** - Medication B consisting of composition 3 (Example 3);
**IV** - intravenous injection;
**IM** - intramuscular injection;
**spray** - intranasal spray.

Preferably, the nasal pharmaceutical composition of the present invention is a liquid dosage form prepared in the form of a nasal spray. The expression "a liquid dosage form" includes a solution, an emulsion, or a suspension intended for nasal administration (delivery to the nasal mucosa).

The pharmaceutical composition of the present invention may comprise the active pharmaceutical ingredient in an amount of from about 2.5% w/w to about 20% w/w, preferably, from about 2.5% w/w to about 10% w/w, or, more preferably, from about 2.5 % w/w to about 5% w/w. In one embodiment of the present invention, the active pharmaceutical ingredient may be present in an amount of from about 3 % w/w to about 4% w/w.

The pharmaceutical composition of the present invention may ensure the delivery of nalbuphine as the base and/or salts thereof in an amount (per dose) from about 3 mg to about 20 mf, preferably, from about 3 mg to about 10 mg, or, more preferably, from about 3 mg to about 5 mg.

According to one aspect of the present invention, from one to four doses of the nasal nalbuphine-based pharmaceutical composition may be administered to a patient. According to another aspect of the present invention, one dose may ensure the delivery from about 40 µL to about 200 µL (from about 4 mg to about 20 mg) of the pharmaceutical composition. According to yet another aspect of the present invention, one dose may ensure the delivery of about 100 µL of the pharmaceutical composition. In some embodiments of the present invention, one dose may ensure the delivery from about 70 µL to about 140 µL of the pharmaceutical composition.

According to one aspect of the present invention, the pharmaceutical composition is used from 4 to 6 times per day for 3 to 5 days.

The solvents and co-solvents may have a nominal molar weight of between 200 g/mol and 600 g/mole.

The taste, color, and odor correctives may include without limitation: pigments and dyes, organic acids, such as, for example, succinic acid, citric acid, and/or lactic acid; sweeteners such as, for example, saccharin, aspartame, sucralose, cyclamates; natural essential oils; and any artificial flavors.

The nasal pharmaceutical composition of the present invention may be packaged, for example, into Gerresheimer, Schott or Nipro glass vials, 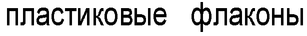 Gerresheimer or AG&K plastic vials, or into pharmaceutically acceptable package, vessel, or vial of any type whatsoever.

As dispensing devices for the nasal pharmaceutical composition of the present invention, Aptar or Ursatec nasal dropper or spray pump systems, for example, with dose volumes of, for example, 50, 70, 100, or 140 µL, or any pharmaceutically acceptable applicators and/or dispensers may be used.

The nasal pharmaceutical composition of the present invention will now be described using the following non-limiting examples.

### Example 1

To a 8 L glass apparatus provided with a stirrer, a thermometer, and a bubbler, 2.5 L of purified water (the solvent) and accurately weighed amount of propylene glycol (the co-solvent) were charged, and 150 g of nalbuphine as the base (the active pharmaceutical ingredient) were added while stirring.

Then the accurately weighed amount of citric acid was added, while stirring, to the solution, and the resulting solution was mixed thoroughly till the complete dissolution of nalbuphine. Then, by adding 1M disodium phosphate (the pH regulator), pH 5.5 was established. Then Kollidone K30, methylparaben, and propylparaben were added, while stirring, the completeness of solution of each component being controlled before the next component is added. The volume of the solution was diluted to 6 L with purified water and mixed thoroughly (composition 1). Composition 1 comprised:

| Active Pharmaceutical Ingredient | Nalbuphine (Base) | 150 g |
|---|---|---|
| pH regulator | Citric acid | 100 g |
| pH regulator | Disodium phosphate | To pH 5.5 |
| Co-solvent | Propylene glycol | 2000 g |
| Viscosity regulator | Kollidone K30 | 140 g |
| Antimicrobial agent | Methylparaben | 5 g |
| Antimicrobial agent | Propylparaben | 1.5 g |
| Solvent | Purified water | Q.S. to 6 L |

Composition 1 was filled into 10-mL colorless glass vials and closed with 120-µL nasal spray pumps.

### Example 2

To a 8 L glass apparatus provided with a stirrer, a thermometer, and a bubbler, 4 L of purified water were charged and 178.6 g of nalbuphine hydrochloride were dissolved therein while stirring.

Then the accurately weighed amounts of chitosan, glutamic acid, propylene glycol, Kollidone K30, and benzalkonium chloride were added, while stirring, to the solution, the completeness of solution of each component being controlled before the next component is added. The volume of the solution was diluted to 5 L with purified water and mixed thoroughly (composition 2). Composition 2 comprised:

| Active Pharmaceutical Ingredient | Nalbuphine hydrochloride | 178.6 g |
|---|---|---|
| Adsorption enhancer | Chitosan | 8.9 g |
| pH regulator | Glutamic acid | 8.9 g |
| Co-solvent | Propylene glycol | 100 g |
| Viscosity regulator | Kollidone K30 | 40 g |
| Antimicrobial agent | Benzalkonium chloride | 0.5 g |
| Solvent | Purified water | Q.S. to 5 L |

Composition 2 was poured under argon into 5-mL amber-colored glass vials and closed with 140-µL nasal spray pumps.

A comparative study of the bioavailability of nalbuphine in the form of a nasal spray at a dose rate of 10 mg (composition 2) versus intravenous and intramuscular injections containing 10 mg of nalbuphine was conducted.

The pharmacokinetics of composition 2 in the form of an intranasal spray, the absolute bioavailability of nalbuphine in the form of a nasal spray, as well as the relative bioavailability of nalbuphine in the form of a nasal spray versus intravenous and intramuscular injections were assessed the compositions being administered under fasting conditions.

The assessment of the comparative safety of nalbuphine was performed following the administration of 10 mg of nalbuphine in different dosage forms (in the form of a intranasal spray versus intravenous and intramuscular injections at a dose rate of 10 mg). The safety assessment was based on the analysis and comparison of adverse reactions which occurred following the administration of nalbuphine in different dosage forms; on the evaluation of vital parameters and the general state of volunteers; and on laboratory analyses.

The study was a single-dose, open-label, randomized, three-period, crossover study of 10 mg of nalbuphine (intranasally, intravenously, and intramuscularly) under fasting conditions. The total number of healthy adult male subjects enrolled was twelve. Throughout the study, the conditions of their hospital stay, a dietary pattern, a water consumption regimen, a physical activity regimen for the volunteers were standardized. The clinical step of study consisted of a screening, two study periods, a washout period, and the final examination. The total duration of study, starting from the screening through the completion of study, was approximately three weeks with a seven-day wash-out period between medication administrations. Each study period was preceded by inpatient hospitalization in the evening of the previous day. The medications were dosed on Day 1 of each study period starting from 08:00 a.m. The subjects stayed in the centre for 24 hours after the administration of the medication under study in each study period. In each study period the following was done: the intake of the medications under study whose prescription was determined by a simple randomization method; blood sampling for the determination of active pharmaceutical ingredient concentration; the measurement of vital data such as blood pressure (BP), heart rate; and questionnaire survey of volunteers.

Blood was sampled as follows: 0 hours, 15 min., 30 min., 45 min., 1 hour, 1 hour 30 min., 2 hours, 2 hours 30 min., 3 hours, 4 hours, 6 hours, 8 hours, 12 hours, and 24 hours post-dosing. In the event of intravenous injection, additional samples were taken 5 min. and 10 min. post-dosing. The actual sampling time was recorded.

The plasma nalbuphine concentration was measured using a validated bioanalytical method and in accordance with the standard operating procedures (SOP) and guidelines. The validated range of detection of nalbuphine in human plasma was from 1.00 to 360.03 ng/mL.

The pharmacokinetic parameters were calculated using noncompartmental pharmacokinetic analysis utilizing a licensed version of Phoenix WinNonLin 8.1 (Pharsight Corp., Certara L.P., USA). Based on the results of assay for nalbuphine in the blood plasma of healthy volunteers, the following pharmacokinetic parameters were calculated:
AUC₍₀₋ₜ₎ is the area under the plasma concentration-time curve (AUC) from time zero (dosing time) to the last time point, *t* [ng×h/mL].
AUC_{(0-∞)} is AUC_{0-*t*} plus the extrapolated AUC from time *t* to infinity [ng×h/mL]. The extrapolation is calculated from the last measured concentration (Cₜ): AUC_{(0-∞)} = AUC₍₀₋ₜ₎ + Cₜ / Kₑₗ.
Cₘₐₓ is the maximum plasma concentration [ng/mL].
Tₘₐₓ is the time to the maximum plasma concentration (if more than one maximum concentration, the time to the first of them) [h].
λ_{z} (Kₑₗ) is the first-order elimination rate constant, which describes the final elimination phase [h]⁻¹.
T_{1/2} is the elimination half-life [h]: T_{1/2} = ln(2)/Kₑₗ.

For each subject, the plasma nalbuphine concentration-time curves were plotted in both direct coordinates and semilog coordinates (combined for all the medications under study in the same coordinate grid). The mean plasma nalbuphine concentration-time curves were also plotted for the medications under study in both direct coordinates and semilog coordinates. For each subject, the actual time intervals of blood sampling were employed.

For all the pharmacokinetic parameters, the following descriptive statistical parameters were calculated: the number of measurements (n), the arithmetic mean value (Mean), the standard deviation (SD), the coefficient of variation (%CV), the minimum value (Min), the maximum value (Max), and the median (Median). In addition, geometric means for AUC₍₀₋ₜ₎, AUC_{(0-∞)}, and Cₘₐₓ were calculated.

On Day 1 of each period, immediately prior to dosing, the volunteers cleaned their noses with a napkin.

Within 30 minutes before intranasal administration, the vial with the pharmaceutical composition provided in the form of spray was prepared for use via spraying five times in accordance with the sponsor's directions regarding vial preparation.

The medications were dosed in the prone position; the volunteer's body was at an angle of 45° relative to the bed. Responsible Investigator administered the amount required of sprayed doses in accordance with the randomization scheme. The volunteers remained in the prone position for 15 minutes post dosing.

Intravenous and intramuscular administrations were performed by medical personnel in accordance with the randomization scheme. The volunteers remained in the prone position for 15 minutes post dosing. 5 minutes after dosing, a study nurse took blood samples from the volunteers after intravenous administration in the prone position.

After the administration of medication, the subjects were proposed to fill in a questionnaire, which contained the following questions:
- Feelings at the administration site;
- Changes in the general state after administration;
- Time points of these changes (commencement and cessation);
- Time point of return to the initial state.

Prior to the study, the volunteers were invited to the investigation site where a study physician informed them about study conduct circumstances. The study physician provided each potential study subject - volunteer - with written information about study.

Each volunteer had enough time to study the information for a volunteer and to make decision. Then, in order to take part in the study, a volunteer signed and dated an Informed Consent Form in two counterparts. One counterpart was issued to the volunteer and the other is kept with Responsible Investigator.

The volunteers came to a screening visit under fasting conditions with a container with a morning urine portion. The screening included the following procedures:
- Fluorography examination (to be conducted not more than 10 months before enrollment);
- Demographic information and anthropometric data such as age, sex, height, weight;
- Calculation of body mass index (BMI);
- MHx (medical history);
- Physical examination;
- Vital data: measuring heart rate, blood pressure, body temperature;
- 12-lead ECG;
- CBC (complete blood count);
- Biochemical analysis of blood.
- Express diagnostics of HIV infection, syphilis, hepatitis B/C markers.
- Common urine examination.
- Breath test for alcohol; and
- Urine narcotic test.

Based on the screening results, Responsible Investigator checked whether or not each volunteer complied with the requirements of the enrollment criteria and made decision on the possibility of his further participation in the clinical study, following which the volunteer was assigned a randomization number.

Each study period was preceded by inpatient hospitalization in the evening of the previous day. On Day 0 (the day before dosing), the subjects (volunteers) were admitted to the hospital inpatient department, where all the required procedures were performed such as physical examination; measuring vital data such as heart rate, blood pressure, body temperature; breath test for alcohol; and urine narcotic test. The fasting period before dosing was not less than 10 hours.

On Day 1 of each study period prior to dosing, IV catheters were installed in the volunteers and the first blood sample was taken.

The medications were dosed on Day 1 of each study period starting from 08:00 a.m. 10 mg of nalbuphine were administered to the volunteers in each case. The route of administration was determined by the randomization scheme. During 4 hours post dosing, food intake was prohibited.

The study was conducted with the involvement of 12 healthy male volunteers. All the healthy volunteers completed the study in full. The demographic information of volunteers is presented in Table 1 below.

**Table 1**

| | **Age** | **Body weight, kg** | **Height, M** | **BMI** |
|---|---|---|---|---|
| N | 12 | 12 | 12 | 12 |
| Mean | 36.67 | 80.45 | 177.38 | 25.7 |
| SD | 5.66 | 6.71 | 6.73 | 2.9 |
| Min | 29.00 | 67.00 | 171.00 | 21.2 |
| Max | 46.00 | 88.70 | 191.50 | 30.0 |

The results of assay for nalbuphine in blood plasma of all the healthy volunteers were statistically processed. To calculate the pharmacokinetic parameters, actual time values were used.

Human plasma nalbuphine concentration was determined in a bioanalytical laboratory. The analysis was performed using AB SCIEX API-4000 LC/MS/MS system provided with TurbolonSpray Ion Source. Positive ions were detected in the Sensitivity MRM (Multiple Reaction Monitoring) mode. The assay for analyte was carried out using the liquid-liquid extraction (LLE) technique. Following extraction, 5 µL of aliquots were entered into the LC/MS/MS system. The data obtained were processed using AB SCIEX Analyst, version 1.6.2. In order to ensure the best fitting of the calibration curve to the data available, a 1/x² weighted linear regression was applied. The lower level of quantification (LLOQ) was 1.00 ng/mL while the upper level of quantification (ULOQ) was 360.03 ng/mL for nalbuphine. The standards for plotting the calibration curve and quality control (QC) samples for nalbuphine met the acceptance criteria for the series of analyses conducted to obtain the final data this indicating a satisfactory performance of the method in the process of the analysis of samples under study obtained from the subjects.

The pharmacokinetic parameters were calculated using a licensed version of Phoenix WinNonLin 8.1 (Pharsight Corp., Certara L.P.,U.S.A.), developed specially for the analysis of pharmacokinetic data. The pharmacokinetic parameters were calculated using noncompartmental pharmacokinetic analysis.

**Fig. 1** is a graphic representation of the mean concentrations of nalbuphine in blood plasma versus time after the administration of 10 mg of nalbuphine in different dosage forms (intranasally, intravenously, intramuscularly).

Table 2 below shows the descriptive statistics of pharmacokinetic parameters obtained for each route of administration of nalbuphine obtained using noncompartmental pharmacokinetic analysis.

For Cₘₐₓ, AUC₍₀₋ₜ₎, Kₑₗ, Table 2 shows the arithmetic mean values ± the standard deviation; for Tₘₐₓ, T_{1/2}, the medians (the minimum and maximum values) are presented.

The absolute bioavailability of nalbuphine following intranasal administration was 50 %.

**Table 2**

| **Pharmacokinetic Parameter** | **Route of Administration** | | |
|---|---|---|---|
| | **Spray** | **IV** | **IM** |
| Cₘₐₓ (ng/mL) | 10.24±3.60 | 50.78±19.54 | 36.43±12.50 |
| Tₘₐₓ (h) | 0.750 (0.500-3.000) | 0.083 (0.083-0.083) | 0.250 (0.250-0.750) |
| AUC₍₀₋ₜ₎ (ng×h/mL) | 41.73±16.69 | 91.08±23.53 | 97.27±23.93 |
| AUC_{(0-∞)} (ng×h/mL) | 48.98±15.24 | 97.69t23.43 | 103.24±23.53 |
| Kₑₗ (h⁻¹) | 0.24925±0.06 | 0.26814±0.04 | 0.27614±0.03 |
| T_{1/2} (h) | 2.741 (2.144-7.206) | 2.629 (2.019-3.358) | 2.422 (2.200-3.112) |

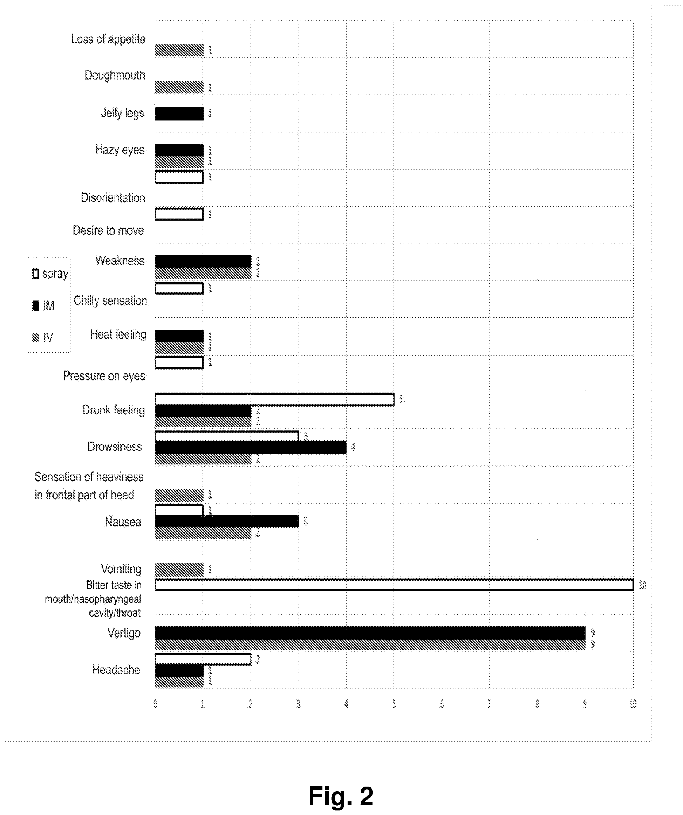

In this study, 25 cases of adverse reactions were recorded after intravenous administration, 24 cases of adverse reactions were recorded after intramuscular administration, and 25 cases of adverse reactions were recorded after intranasal administration of 10 mg of nalbuphine. **Fig. 2** is a comparative diagram of the adverse reactions recorded. Since 40% of adverse reactions after the administration of the intranasal spray (bitter taste intramuscular mouth, nasopharyngeal cavity, throat) were associated with the route of administration itself and lasted for not more than 15 minutes, these adverse reactions do not affect the safety of this route of administration. The intranasal spray is, thus, two-fold more safe than intramuscular administration and intravenous administration this also being an advantage of the present invention.

The absolute bioavailability of the intranasal form of nalbuphine is about 50%. Following the administration of intranasal spray, adverse reactions occur half as many as compared with those after intramuscular administration and intravenous administration at the same dose rate.

### Example 3

Also, in accordance with the present invention, composition 3 was produced as follows:

| Active Pharmaceutical Ingredient | Nalbuphine hydrochloride | 178.6 g |
|---|---|---|
| Adsorption enhancer | Chitosan | 89.3 g |
| pH regulator | Glutamic acid | 89.3 g |
| Antimicrobial agent | Benzalkonium chloride | 0.5 g |
| Solvent | Purified water | Q.S. to 5 L |

To a 8 L glass apparatus provided with a stirrer, a thermometer, and a bubbler, 4 L of purified water were charged and 178.6 g of nalbuphine hydrochloride were dissolved therein while stirring. Then the accurately weighed amounts of chitosan, glutamic acid, and benzalkonium chloride were added, while stirring, to the solution, the completeness of solution of each component being controlled before the next component is added. The volume of the solution was diluted to 5 L with purified water and mixed thoroughly (composition 3)

Composition 3 was poured under argon into 5-mL glass vials and closed with 100-µL nasal spray pumps.

### Comparative pharmacokinetic study of pharmaceutical compositions 2 and 3. Assessment of different amounts of chitosan to the bioavailability of nalbuphine in the form of nasal spray at a dose rate of 7 mg

In this study, the effect of chitosan of the pharmacokinetics of the new dosage form in the form of intranasal spray was assessed with administration being performed under fasting conditions.

Additional objectives were to assess comparative safety of nalbuphine following intranasal administration of 7 mg of nalbuphine with different amounts of chitosan as an auxiliary ingredient. The safety assessment was based on the analysis and comparison of adverse reactions which occurred following the administration of nalbuphine; on the evaluation of vital parameters and the general state of volunteers; and on laboratory analyses.

The study was a single-dose, open-label, randomized, two-period, crossover study of 7 mg of nalbuphine in the form of intranasal spray under fasting conditions. Six healthy volunteers, who took part also in the study described in Example 2, participated in this study. Throughout the study, the conditions of their hospital stay, a dietary pattern, a water consumption regimen, a physical activity regimen for the volunteers were standardized. The clinical step of study consisted of a screening, two study periods, a washout period, and the final examination. The total duration of study, starting from the screening through the completion of study, was approximately two weeks with a seven-day wash-out period between medication administrations. Each study period was preceded by inpatient hospitalization in the evening of the previous day. The medications were dosed on Day 1 of each study period starting from 08:00 a.m. The subjects stayed in the centre for 24 hours after the administration of the medication under study under study in each study period. In each study period the following was done: the intake of the medications under study whose prescription was determined by a simple randomization method; blood sampling for the determination of active pharmaceutical ingredient concentration; the measurement of vital data such as blood pressure (BP), heart rate; and questionnaire survey of volunteers.

The medications under study:
A - Composition 2 (Example 2); and
B - Composition 3.

Blood was sampled as follows: 0 hours, 15 min., 30 min., 45 min., 1 hour, 1 hour 30 min., 2 hours, 2 hours 30 min., 3 hours, 4 hours, 6 hours, 8 hours, 12 hours, and 24 hours post-dosing. The actual sampling time was recorded.

The plasma nalbuphine concentration was measured using a validated bioanalytical method and in accordance with the standard operating procedures (SOP) and guidelines. The validated range of detection of nalbuphine in human plasma was from 1.00 to 360.03 ng/mL.

The pharmacokinetic parameters were calculated using noncompartmental pharmacokinetic analysis utilizing a licensed version of Phoenix WinNonLin 8.1 (Pharsight Corp., Certara L.P., USA). Based on the results of assay for nalbuphine in the blood plasma of healthy volunteers, the following pharmacokinetic parameters were calculated:
AUC₍₀₋ₜ₎ is the area under the plasma concentration-time curve (AUC) from time zero (dosing time) to the last time point, *t* [ng×h/mL].
AUC_{(0-∞)} is AUC_{0-*t*} plus the extrapolated AUC from time *t* to infinity [ng×h/mL]. The extrapolation is calculated from the last measured concentration (Cₜ): AUC_{(0-∞)} = AUC₍₀₋ₜ₎ + Cₜ / Kₑₗ.
Cₘₐₓ is the maximum plasma concentration [ng/mL].
Tₘₐₓ is the time to the maximum plasma concentration (if more than one maximum concentration, the time to the first of them) [h].
λ_{z} (Kₑₗ) is the first-order elimination rate constant, which describes the final elimination phase [h]⁻¹.
T_{1/2} is the elimination half-life [h]: T_{1/2} = ln(2)/Kₑₗ.

For each subject (volunteer), the plasma nalbuphine concentration-time curves were plotted in both direct coordinates and semilog coordinates (combined for all the medications under study in the same coordinate grid). The mean plasma nalbuphine concentration-time curves were also plotted for the medications under study in both direct coordinates and semilog coordinates. For each subject, the actual time intervals of blood sampling were employed.

For all the pharmacokinetic parameters, the following descriptive statistical parameters were calculated: the number of measurements (n), the arithmetic mean value (Mean), the standard deviation (SD), the coefficient of variation (%CV), the minimum value (Min), the maximum value (Max), and the median (Median). In addition, geometric means for AUC₍₀₋ₜ₎, AUC_{(0-∞)}, and Cₘₐₓ were calculated.

On Day 1 of each period, immediately prior to dosing, the volunteers cleaned their noses with a napkin.

Within 30 minutes before intranasal administration, the vial with the pharmaceutical composition provided in the form of spray was prepared for use via spraying five times in accordance with the sponsor's directions regarding vial preparation.

The medications were dosed in the prone position; the volunteer's body was at an angle of 45° relative to the bed. Responsible Investigator administered the amount required of sprayed doses in accordance with the randomization scheme. The volunteers remained in the prone position for 15 minutes post dosing.

After the administration of medication, the subjects were proposed to fill in a questionnaire, which contained the following questions:
- Feelings at the administration site;
- Changes in the general state after administration;
- Time points of these changes (commencement and cessation);
- Time point of return to the initial state.

Each study period was preceded by inpatient hospitalization in the evening of the previous day. On Day 0 (the day before dosing), the subjects (volunteers) were admitted to the hospital inpatient department, where all the required procedures were performed such as physical examination; measuring vital data such as heart rate, blood pressure, body temperature; breath test for alcohol; and urine narcotic test. The fasting period before dosing was not less than 10 hours.

On Day 1 of each study period prior to dosing, IV catheters were installed in the volunteers and the first blood sample was taken.

The medications were dosed on Day 1 of each study period starting from 08:00 a.m. 7 mg of nalbuphine were administered to the volunteers in each period. The medication to be studied was determined by the randomization scheme. During 4 hours post dosing, food intake was prohibited.

### Distribution of the subjects (volunteers)

The study was conducted with the involvement of 6 healthy male volunteers. All the healthy volunteers completed the study in full.

Six subjects were enrolled, and all the subjects were healthy adult male volunteers. All the six subjects completed in full their involvement in the study.

The results of assay for nalbuphine in blood plasma of all the healthy volunteers were statistically processed. To calculate the pharmacokinetic parameters, actual time values were used.

Human plasma nalbuphine concentration was determined in a bioanalytical laboratory. The analysis was performed using AB SCIEX API-4000 LC/MS/MS system provided with TurbolonSpray Ion Source. Positive ions were detected in the Sensitivity MRM (Multiple Reaction Monitoring) mode. The assay for analyte was carried out using the liquid-liquid extraction (LLE) technique. Following extraction, 5 µL of aliquots were entered into the LC/MS/MS system. The data obtained were processed using AB SCIEX Analyst, version 1.6.2. In order to ensure the best fitting of the calibration curve to the data available, a 1/x² weighted linear regression was applied. The lower level of quantification (LLOQ) was 1.00 ng/mL while the upper level of quantification (ULOQ) was 360.03 ng/mL for nalbuphine. The standards for plotting the calibration curve and quality control (QC) samples for nalbuphine met the acceptance criteria for the series of analyses conducted to obtain the final data this indicating a satisfactory performance of the method in the process of the analysis of samples under study obtained from the subjects.

The pharmacokinetic parameters were calculated using a licensed version of Phoenix WinNonLin 8.1 (Pharsight Corp., Certara L.P.,U.S.A.), developed specially for the analysis of pharmacokinetic data. The pharmacokinetic parameters were calculated using noncompartmental pharmacokinetic analysis.

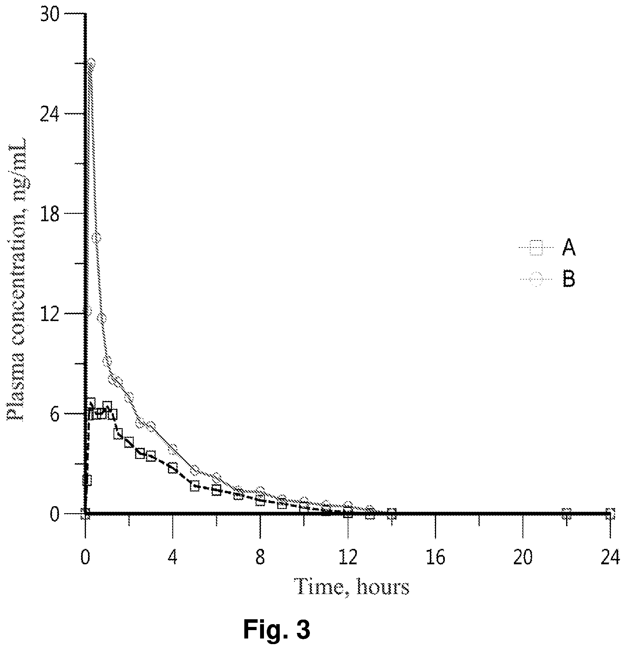

**Fig. 3** is a graphic representation of the mean concentrations of nalbuphine in blood plasma versus time after the administration of 7 mg of nalbuphine with different amounts of chitosan.

Table 3 below shows the descriptive statistics of pharmacokinetic parameters for the medications under study obtained using noncompartmental pharmacokinetic analysis.

**Table 3**

| **Pharmacokinetic Parameter** | **Medication** | |
|---|---|---|
| | **A** | **B** |
| Cₘₐₓ (ng/mL) | 7.39t3.28 | 28.93±15.01 |
| Tₘₐₓ (h) | 0.500 (0.250-1.000) | 0.167 (0.167-0.250) |
| AUC₍₀₋ₜ₎ (ng×h/mL) | 24.96±10.44 | 45.84±16.27 |
| AUC_{(0-∞)} (ng×h/mL) | 27.72±10.11 | 48.14±16.63 |
| Kₑₗ (h⁻¹) | 0.26295±0.02 | 0.27228 ±0.05 |
| T_{1/2} (h) | 2.594 (2.396-2.914) | 2.549 (1.963-3.559) |

For Cₘₐₓ, AUC₍₀₋ₜ₎, Kₑₗ, Table 3 shows the arithmetic mean values ± the standard deviation; for Tₘₐₓ, T_{1/2}, the medians (the minimum and maximum values) are presented.

Following the administration of spray with the elevated amount of chitosan, the resulting bioavailability is about two-fold bioavailability after the administration of medication A. The elevation of the amount of chitosan as an auxiliary ingredient of spray results, thus, in a significant increase in the bioavailability of spray. The absolute bioavailability of nalbuphine after intranasal administration of medication B is about 80 %.

In this study, there was no adverse reaction in any of the study periods. The safety of medication A is, thus, similar to that of medication B.

The elevation of the amount of chitosan in the spray dosage form increases the bioavailability of nalbuphine virtually by a factor of two and, at the same time, speeds up the achievement of maximum plasma concentration this in turn improving the efficacy of nalbuphine in the form of intranasal spray.

The present invention is not limited by the above exemplary embodiments.

This specification contains particulars which are required and sufficient to understand clearly the essence of the present invention. Particulars, which are obvious to those with ordinary skill in the art and which do not promote to a better understanding of the present invention, are omitted.

For example, in accordance with the present invention, an embodiment is possible wherein, in order to improve a long-term stability of nalbuphine, the pharmaceutical composition may be a two-component system comprising an active pharmaceutical ingredient and a solvent with an adsorption enhancer and other auxiliary ingredients such as a pH regulator, an enhancer of the solubility of the active pharmaceutical ingredient, etc. The active ingredient is in solid phase, for example, powder, granules, tablets, pellets. Prior to use, the user dissolves the active pharmaceutical ingredient in the solvent. The solvent may already be within a dispenser.

### Technical Result

The technical result of the present invention is the provision of a nasal pharmaceutical composition based on nalbuphine and/or salts thereof having a high bioavailability of nalbuphine to treat (relieve) moderate to severe pain.

## Claims

1. A pharmaceutical composition for nasal administration based on nalbuphine and/or salts thereof, **characterized in that** the composition comprises:
- an active pharmaceutical ingredient for which nalbuphine as the base and/or salts thereof are used,
- an enhancer of the adsorption of said active pharmaceutical ingredient with chitosan and/or derivatives thereof including in the form of chitosan salts being used as said adsorption enhancer,
- a solvent of said active pharmaceutical ingredient and said adsorption enhancer,
the amount of the active pharmaceutical ingredient being 2.5 to 20 % *w*/*w* and the active pharmaceutical ingredient to adsorption enhancer ratio being from 1:0.1 through 1:1.

2. The pharmaceutical composition according to claim **1,** ***characterized in that*** salts of nontoxic organic and nonorganic acids such as, for example, acetic acid, benzoic acid, citric acid, fumaric acid, glutamic acid, hydrobromic acid, hydrochloric acid, lactic acid, maleic acid, nitric acid, pantothenic acid, phosphoric acid, succinic acid, sulfuric acid, and tartaric acid, are used as nalbuphine salts.

3. The pharmaceutical composition according to claim **1,** ***characterized in that*** the absolute bioavailability of the active pharmaceutical ingredient is at least 40 %.

4. The pharmaceutical composition according to claim **1,** ***characterized in that*** the composition has a pH of between 4.0 and 7.0.

5. The pharmaceutical composition according to claim **4,** ***characterized in that*** hydrobromic acid, citric acid, lactic acid, acetic acid, malic acid, phosphoric acid, sodium hydroxide, trometamol, diethanolamine, tert-butylamine, tetrahydroxypropyl ethylenediamine (THPE), potassium carbonate, potassium hydroxide, sodium carbonate, sodium citrate, and sodium lactate, for example, are used as pH regulators.

6. The pharmaceutical composition according to claim **1**, ***characterized in that*** it comprises an enhancer of the solubility of the active pharmaceutical ingredient with solubilizers and/or surfactants being used as said solubility enhancer.

7. The pharmaceutical composition according to claim **6**, ***characterized in that*** monoethyl ether of diethylene glycol, cyclodextrins, glyceryl monostearate, lecithin, poloxamer, polyvinyl pyrrolidone, polyethylene alkyl ethers, hydrogenated castor oil derivatives, polyoxyethylene stearates, citric acid and ascorbic acid, polysorbates, sorbitan esters, polyvinyl alcohol, benzalkonium chloride and lauryl sulphate, for example, are used as said solubilizers.

8. The pharmaceutical composition according to claim **6**, ***characterized in that*** polysorbates and macrogols, for example, are used as said surfactants, and poloxamers and povidones are used as said solubilizers.

9. The pharmaceutical composition according to claim **1**, ***characterized in that*** the composition comprises a microbiological stability preservative with, for example, methylparaben, propylparaben, sodium benzoate, benzyl alcohol, benzalkonium chloride, and chlorobutanol being used as said microbiological stability preservative.

10. The pharmaceutical composition according to claim **1**, ***characterized in that*** water or its mixture with organic co-solvents is/are used as said solvent with, for example, ethanol, propylene glycol, glycerol, and polyethylene glycols being used as said co-solvents.

11. The pharmaceutical composition according to claim **1**, ***characterized in that*** the composition comprises a viscosity regulator with, for example, hydroxypropyl methylcellulose, hydroxypropyl cellulose, hydroxyethyl cellulose, methylcellulose, sodium carboxylmethylcellulose, and polyvinyl pyrrolidones of various grades, such as K15, K30, K60, and K90, being used as said viscosity regulator.

12. The pharmaceutical composition according to claim **1**, ***characterized in that*** the composition comprises an osmolarity regulator with, for example, sodium metabisulfite, sodium bisulfite, ethylenediaminetetraacetic acid (EDTA) disodium salt, and ascorbic acid being used as said osmolarity regulator.

13. The pharmaceutical composition according to claim **12**, ***characterized in that*** the osmotic pressure of the pharmaceutical composition is 300 to 700 mOsm/L.

14. The pharmaceutical composition according to claim **1**, ***characterized in that*** the composition comprises an antimicrobial agent with, for example, benzyl alcohol, benzoic acid and/or salts thereof, sorbates, benzalkonium chloride, phenylethyl alcohol, methylparaben, ethylparaben, propylparaben, and butylparaben being used as said antimicrobial agent.

15. The pharmaceutical composition according to claim **1**, ***characterized in that*** the composition comprises taste, color, and odor correctives.

16. A method of using the pharmaceutical composition according to any of claims **1** to **15**, ***characterized in that*** the pharmaceutical composition is administered at a dose rate of between 3 and 20 mg.
